# EUROPÄISCHE PATENTSCHRIFT

(11) **EP 2 714 100 B1**
(45) Veröffentlichungstag und Bekanntmachung des Hinweises auf die Patenterteilung: **11.07.2018**
(21) Anmeldenummer: 12727294.6
(22) Anmeldetag: 29.05.2012
(51) Int. Cl.: A61L 2/03, C12M 1/26

(54) **KOLONIE-VEREINZELUNGSVORRICHTUNG UND VERFAHREN ZUM VEREINZELN VON BAKTERIENKOLONIEN**
COLONY PICKER AND METHOD FOR PICKING BACTERIAL COLONIES
DISPOSITIF D'ISOLEMENT DE COLONIES ET PROCÉDÉ POUR L'ISOLEMENT DE COLONIES BACTÉRIENNES

(30) Priorität: 01.06.2011 DE 102011103784
(43) Veröffentlichungstag der Anmeldung: 09.04.2014
(73) Patentinhaber: Technische Universität Braunschweig Carolo-Wilhelmina, 38106 Braunschweig (DE)
(72) Erfinder: DIETRICH, Franz, 38104 Braunschweig (DE); BURISCH, Arne, 38104 Braunschweig (DE); RAATZ, Annika, 38114 Braunschweig (DE)
(74) Vertreter: Plöger, Jan Manfred
(86) Internationale Anmeldenummer: PCT/EP2012/002264
(87) Internationale Veröffentlichungsnummer: WO 2012/163514

(56) Entgegenhaltungen:
- JP-A- 61 280 264
- US-A- 2 134 378
- US-A- 3 742 187
- US-A- 4 115 200

## Beschreibung

Die Erfindung betrifft eine Kolonie-Vereinzelungsvorrichtung mit (a) zumindest einer Vereinzelungsnadel, (b) einer Haltevorrichtung, in der die zumindest eine Vereinzelungsnadel befestigt ist, und (c) einer Sterilisiervorrichtung, die angeordnet ist zum, insbesondere automatischen Sterilisieren der Vereinzelungsnadel. Gemäß einem zweiten Aspekt betrifft die Erfindung ein Verfahren zum Vereinzeln von Mikroorganismen.

Um neuartige Mikroorganismen aufzufinden werden Proben, beispielsweise Bodenproben, genommen und auf einem Anzuchtmedium, beispielsweise Agar-Agar in einer Petrischale mit einer Impföse in schleifenförmigen Bahnen ausgestrichen. Dadurch werden die Mikroorganismen soweit voneinander separiert, dass in der darauf folgenden Anzucht separate Höfe, die auch als Kolonien bezeichnet werden, wachsen. Da jede Kolonie aus einem einzelnen Organismus durch Teilung entstanden ist, befindet sich in einer Kolonie nur genetisch identisches Material. Diese Reinheit ist für die nachfolgenden Analyseprozesse von entscheidender Bedeutung und muss erhalten werden. Für nachfolgende Analyseprozesse müssen Organismen der einzelnen Höfe auf sterile Nährmedien, die beispielsweise in Mikrotiterplatten vorbereitet bereit stehen, übertragen werden. Dazu werden Kolonie-Vereinzelungsvorrichtungen eingesetzt, die auch als Kolonie-Picker bezeichnet werden.

Derartige Kolonie-Vereinzelungsvorrichtungen besitzen zumindest eine Vereinzelungsnadel, die in eine zuvor ausgewählte Kolonie eingetaucht wird. Beim Eintauchen der Vereinzelungsnadel bleiben einzelne Organismen an der Vereinzelungsnadel hängen. Danach wird die Vereinzelungsnadel in Kontakt mit einem sterilen zweiten Anzuchtmedium gebracht. Mit hoher Wahrscheinlichkeit befinden sich auf dem zweiten Anzuchtmedium dann nur noch genetisch identische Organismen einer Art, die dann untersucht werden können.

Nach jedem Einbringen der Vereinzelungsnadel in die Kolonie und dem nachfolgenden Einbringen in das Anzuchtmedium muss die Vereinzelungsnadel sterilisiert werden. Das geschieht bei bekannten Vereinzelungsvorrichtungen durch Erhitzen mit einer äußeren Wärmequelle (beispielsweise Heißluft, Flamme, erhitzte Glaskugeln), durch chemische oder mechanische Reinigung oder aber eine Kombination hiervon, wobei Hilfsstoffe, wie beispielsweise Reinigungsflüssigkeit oder Brennstoff benötigt werden können. Nachteilig an bekannten Kolonie-Vereinzelungsvorrichtungen ist deren aufwendige Konstruktion.

Aus der US 4,115,200 A ist ein Animpfgerät bekannt, mittels dem ein Inokulat auf eine Mehrzahl an Anzuchtmedien verteilt werden kann. Dazu sind Drahtschleifen vorgesehen, die zunächst in das Inokulat eingetaucht und danach in Kontakt mit einem Nährboden gebracht werden. Zum Desinfizieren können die Schleifen elektrisch erhitzt werden. Ein derartiger Inokulator ist zum Vereinzeln von Kolonien nicht geeignet.

Aus der US 3,742,187 ist ein Inokulator beschrieben, bei dem ein schleifenförmiger elektrischer Leiter mit einem Handteil gehalten werden kann. Auch bei diesem Gerät erfolgt das Sterilisieren durch Erhitzen der Schleife. Nachteilig ist auch an diesem Gerät, dass ein Vereinzeln von Kolonien nicht möglich ist, da die Kontaktfläche, mit der die Schleife in Kontakt mit der Mutterkolonie kommt, so groß ist, dass mit einer sehr hohen Wahrscheinlichkeit. Bakterien mehrerer Kolonien entnommen werden, was unerwünscht ist.

Eine Kolonie-Vereinzelunasvorrichtung ist in der JP 61/280,264 beschrieben. Dieser Druckschrift sind keine Angaben über die Sterilisation zu entnehmen.

Der Erfindung liegt die Aufgabe zugrunde, eine konstruktiv besonders einfache Kolonie-Vereinzelungsvorrichtung anzugeben.

Die Erfindung löst das Problem durch eine gattungsgemäße Kolonie-Vereinzelungsvorrichtung, bei der die Sterilisiervorrichtung eine Stromversorgung, die elektrisch mit der Vereinzelungsnadel verbunden oder verbindbar ist, und eine Elektrode aufweist, wobei die Elektrode automatisch so mit einer Nadelspitze einer Vereinzelungsnadel in Kontakt bringbar angeordnet ist, dass ein elektrischer Strom durch Elektrode und Nadelspitze leitbar ist, der zumindest eine Vereinzelungsnadel zumindest abschnittsweise so stark erwärmt, dass sie sterilisiert wird.

Gemäß eines zweiten Aspektes löst die Erfindung das Problem durch ein Verfahren zum Vereinzeln von Kolonien von Mikroorganismen, insbesondere Bakterien, mit den Schritten (i) Positionieren einer Vereinzelungsnadel einer Kolonie-Vereinzelungsvorrichtung, so dass eine Nadelspitze der Vereinzelungsnadel in eine Kolonie ragt (ii) danach Positionieren der Nadelspitze in einem Anzuchtmedium, so dass die Kolonie dorthin übertragen und somit vereinzelt wird, (iii) danach In-Kontakt-Bringen der Nadelspitze mit einer Elektrode der Kolonie-Vereinzelungsvorrichtung, (iv) Bewirken eines elektrischen Stroms durch Elektrode und Nadelspitze in die Vereinzelungsnadel, so dass sich zumindest die Nadelspitze auf eine Sterilisierungstemperatur erwärmt, und (v) nach einer vorgegebenen Sterilisierzeit Vermindern oder Unterbinden des Stromflusses durch die Vereinzelungsnadel, so dass die Nadelspitze abkühlt.

Vorteilhaft an der Erfindung ist, dass der elektrische Strom die Vereinzelungsnadel an ihrer Nadelspitze besonders stark erwärmt, wenn dort der Querschnitt klein ist. Ein weiterer Vorteil ist es, dass handelsübliche Nadeln, wie sie im medizinischen Bedarf oder im Laborbedarf erhältlich sind, verwendet werden können. So sind zusätzlich an den Seiten der Nadel angebrachte Elektroden entbehrlich. Ein weiterer Vorteil ist es, dass das Sterilisieren rein physikalisch erfolgen kann, das heißt, dass keine Hilfsstoffe, wie zum Beispiel Reinigungsflüssigkeit oder Brennstoff, benötigt werden.

Im Rahmen der vorliegenden Beschreibung wird unter einer Nadel, oder auch Vereinzelungsnadel ein längliches Element verstanden, mittels dem Mikroorganismen oder anderweitiges, flüssiges, gelartiges oder festes Probenmaterial von einem Ort zu einem anderen transferiert wird. Insbesondere handelt es sich bei der Vereinzelungsnadel um eine Vollnadel, also nicht um eine Hohlnadel. Es ist günstig, wenn die Nadel aus einem biokompatiblen Werkstoff besteht, wie beispielsweise eine kobaltfreie Nickellegierung.

Unter der Nadelspitze wird insbesondere der Bereich an der Spitze der Vereinzelungsnadel verstanden.

Unter dem Merkmal, dass ein elektrischer Strom durch Elektrode und Nadelspitze fließt, wird verstanden, dass die Richtung des Stromflusses unerheblich ist. Es ist zudem möglich Gleichstrom, Wechselstrom, eine Folge von Pulsen oder einen anderen zeitlichen Verlauf der Stromstärke zu verwenden. Es ist dabei unerheblich, ob die Stromstärke oder die angelegte Spannung vorgegeben wird, so lange die Sterilisiertemperatur und Sterilisierzeit eingehalten werden.

Unter der Haltevorrichtung wird insbesondere jede Vorrichtung verstanden, an der die Vereinzelungsnadel befestigt ist. Diese Verbindung kann lösbar sein (mit oder ohne zusätzlichem Werkzeug), kann aber auch nicht lösbar, zum Beispiel geklebt sein. Unter dem Merkmal, dass die Elektrode automatisch mit der Nadelspitze in Kontakt bringbar angeordnet ist, wird insbesondere verstanden, dass ein Antrieb vorhanden ist, der aufgrund eines ohne menschlichen Zutuns ablaufenden Programms die Elektrode aus einer ersten Position, in der die Nadelspitze nicht in Kontakt mit der Elektrode ist, in eine zweite Position bringt, in der Elektrode und Nadelspitze Kontakt haben.

Insbesondere umfasst die Kolonie-Vereinzelungsvorrichtung eine Sterilisiervorrichtung, mittels der die Vereinzelungsnadel automatisch oder halbautomatisch in Kontakt mit der Elektrode und außer Kontakt von der Elektrode bringbar ist.

Unter dem Merkmal, dass die Elektrode automatisch mit der Nadelspitze in Kontakt bringbar angeordnet ist, wird insbesondere verstanden, dass entweder ein Antrieb oder aber ein Mechanismus vorhanden ist, der aufgrund eines ohne menschlichen Zutuns ablaufenden Programms die Vereinzelungsnadel und die Elektrode aus einer ersten Position relativ zueinander, in der die Nadelspitze und Elektrode nicht miteinander in Kontakt sind, relativ zueinander in eine zweite Position bringt, in der die Elektrode und die Nadelspitze Kontakt haben. Es ist hierbei unerheblich, durch welche Kinematik diese Bewegung ausgeführt wird (entweder Bewegung der Elektrode oder Bewegung der Nadel oder beides), vielmehr ist die relative Positionierung zueinander entscheidend. Unter dem genannten Mechanismus wird eine kinematische Anordnung verstanden, bei der externe Antriebe einen Mechanismus zur Bewegung der Vereinzelungsnadel betätigen, im Rahmen eines ohne menschliches Zutun ablaufenden Programms.

Unter externen Antrieben werden hier Antriebe verstanden, die nicht Bestandteil der Sterilisiervorrichtung oder des Revolverkopfes sind. Beispielsweise werden die Antriebe der Positioniervorrichtung als externe Antriebe benutzt, um aufgrund eines ohne menschlichen Zutuns ablaufenden Programms den Revolverkopf entlang eines fest stehenden Nockens zu führen, um den Revolverkopf weiter zu drehen.

Unter halbautomatischer Betätigung wird verstanden, dass der Vorgang bei dem der Kontakt hergestellt wird, oder der Vorgang bei dem der Kontakt gelöst wird, durch menschliches Zutun, beispielsweise durch Befehlseingabe, durch Betätigung eines Schalters oder durch mechanische Betätigung ausgelöst wird.

Gemäß einer bevorzugten Ausführungsform besitzt die Kolonie-Vereinzelungsvorrichtung eine Positioniervorrichtung, die die Vereinzelungsnadel, den Träger der Kolonie (zum Beispiel Petri-Schale) und den Träger des Anzuchtmediums (zum Beispiel Mikrotiterplatte) relativ zueinander positioniert. Gemäß einer bevorzugten Ausführungsform stehen der Träger der Kolonie und der Träger des Anzuchtmediums fest relativ zur Basis der Positioniervorrichtung. Weiterhin hat diese Positioniervorrichtung drei translatorische Freiheiten zur Positionierung und trägt die Sterilisiervorrichtung und die Haltevorrichtung am Endeffektor. Gemäß einer bevorzugten Ausführungsform ist die Positioniervorrichtung so ausgeführt, dass eine Hubachse für die Vertikalbewegung und eine ebene Fünfgelenk-Parallelkinematik für die Horizontalbewegung kinematisch seriell angeordnet sind.

Vorzugsweise umfasst die Haltevorrichtung eine Schwenk- oder Verschiebevorrichtung, bevorzugt einen Revolver. An der Haltevorrichtung können mehrere Vereinzelungsnadeln befestigt sein. Eine Schwenkvorrichtung besitzt eine oder mehrere Drehachsen, um die eine oder mehrere Vereinzelungsnadel(n) geschwenkt werden können. Eine Verschiebevorrichtung besitzt eine oder mehrere Linearführungen, entlang derer eine oder mehrere Vereinzelungsnadeln verschoben werden können.

Gemäß einer bevorzugten Ausführungsform umfasst die Haltevorrichtung eine Kontaktiervorrichtung, die den Revolver dreht und die Nadelspitze auf die Elektrode zu und von dieser weg bewegt. Ein Ausschwenken einer Vereinzelungsnadel aus der im Vereinzelungsprozess aktiven Stellung, der Prozess-Stellung ist damit gleichzeitig ein Schwenkvorgang mindestens einer Nadel auf die Elektrode zu oder von dieser weg. Diese Ausführung hat beispielsweise vier Vereinzelungsnadeln. In dieser Ausführung ist die im Vereinzelungsprozess aktive Nadel senkrecht angeordnet. Zuerst wird mittels der ersten Vereinzelungsnadel Probenmaterial übertragen. Dann wird der Revolver gedreht, so dass die erste Nadel aus der Prozessstellung herausgeschwenkt wird und stattdessen eine zweite, sterile Vereinzelungsnadel aus einer Warteposition in die Prozessstellung geschwenkt wird. Diese zweite Nadel steht damit für den nächsten Vereinzelungszyklus bereit. Die unsterile erste Nadel wurde bei dem beschriebenen Schwenkvorgang in die Sterilisierstellung geschwenkt, in Kontakt mit der Elektrode gebracht und wird von der Sterilisiereinrichtung sterilisiert. Sie kann abkühlen, beispielsweise in einer Wartestellung, bevor sie nach zumindest einem weiteren Schwenkvorgang des Revolvers erneut in die Prozessstellung eingeschwenkt wird.

Vorzugsweise sind die Elektrode und die Nadel auch mit einer Vorrichtung zur Anwesenheitsprüfung verbunden oder verbindbar. Diese Vorrichtung zur Anwesenheitsprüfung umfasst beispielsweise eine elektrische Schaltung zur Durchgangsprüfung, die über einen sehr kleinen Sensorstrom die Anwesenheit, beziehungsweise das korrekte Einschwenken der Nadel prüft. Die Elektrode ist damit gleichzeitig auch als ein Sensorelement zu verstehen. Ein kleiner Sensorstrom verhindert eine Funkenbildung beim Einschwenken der Nadel. Die mit der Anwesenheitsprüfungsvorrichtung und der Sterilisiereinheit verbundene Steuereinheit bewirkt dann automatisch ohne weiteres menschliches Zutun nach einer Wartezeit T_w den Strom zur Sterilisierung durch die Elektrode und die Nadel.

Am Revolver kann eine Mehrzahl an Vereinzelungsnadeln befestigt sein, beispielsweise zumindest vier. Ist mittels der ersten Vereinzelungsnadel Bakterienmaterial übertragen worden, so wird der Revolver gedreht, so dass eine weitere Vereinzelungsnadel zum Eintauchen in Primär-Bakterienkolonie zur Verfügung steht. Die nicht mehr sterile erste Vereinzelungsnadel wird in der Zwischenzeit in Kontakt mit der Elektrode gebracht und sterilisiert. Sie kann abkühlen, bevor sie nach zumindest einer weiteren Drehung des Revolvers erneut zum Einsatz kommt.

Vorzugsweise ist die Elektrode fest relativ zur Basis der Positioniereinrichtung oder fest relativ zur Haltevorrichtung angeordnet. In der bevorzugten Anordnung in Form des Revolvers ist die Fixierung fest relativ zum Revolver, also am Endeffektor der Positioniervorrichtung, bevorzugt.

Vorzugsweise besitzt der Revolver einen Sitz und einen am Sitz drehbar gelagerten Revolverkopf, wobei die Vereinzelungsnadel am Revolverkopf befestigt ist. Vorzugsweise ist dann die Elektrode fest relativ zum Sitz befestigt, so dass sich die Elektrode mitbewegt, wenn die Vereinzelungsnadel sich auf die Primär-Bakterienkolonie zu und von dieser wieder weg bewegt.

Vorzugsweise ist die Elektrode federnd ausgebildet und/oder gelagert. Beispielsweise kann die Elektrode dadurch federnd ausgebildet sein, dass sie zumindest an einer Stelle so dünn ist, dass sie als Blattfeder wirkt, wobei die Federkonstante so klein ist, dass auf die Nadelspitze nur eine so kleine Kraft ausgeübt wird, dass die Nadelspitze stets plastisch unverformt bleibt. Alternativ oder zusätzlich kann die Elektrode federnd gelagert sein, das heißt, dass es möglich, nicht aber notwendig ist, dass die Elektrode selbst federnd ist. Es ist vielmehr möglich, dass eine Elektrodenaufnahme der Elektrode federnd ist. Auch in diesem Falle ist eine Federkonstante, der Aufhängung so klein gewählt, dass eine hinreichend kleine Federkraft auf die Nadelspitze der Vereinzelungsnadel ausgeübt wird. Insbesondere ist die Elektrode so federnd ausgebildet und/oder gelagert, dass die Nadelspitze unverformt bleibt, wenn sie durch den fließenden Strom erhitzt wird.

Vorzugsweise besitzt die Kolonie-Vereinzelungsvorrichtung eine Steuereinheit, die ausgewählt ist zum automatischen Durchführen eines Verfahrens mit den Schritten (i) Positionieren der Vereinzelungsnadel, so dass die Nadelspitze in eine Kolonie ragt, (ii) danach Positionieren der Nadelspitze in einem Anzuchtmedium, so dass die Kolonie übertragen wird, (iii) danach In-Kontakt-Bringen der Nadelspitze mit der Elektrode, (iv) Bewirken eines elektrischen Stroms durch Elektrode und Nadelspitze in die Vereinzelungsnadel, so dass sich die Vereinzelungsnadel auf eine Sterilisierungstemperatur erwärmt, und (v) nach einer vorgegebenen Sterilisierzeit Vermindern oder Unterbinden des Stromflusses durch die Vereinzelungsnadel, so dass die Vereinzelungsnadel abkühlt.

Vorzugsweise ist die Positioniervorrichtung ausgebildet zum Positionieren der Nadelspitze auf eine vorgegebene Raumposition. Hierunter ist insbesondere zu verstehen, dass eine Raumposition innerhalb eines dreidimensionalen Arbeitsraums vorgebbar ist. Es ist insbesondere ein automatischer oder halbautomatischer Betrieb möglich, bei dem eine Kolonie mehrfach angefahren und auf eine Vielzahl von Anzuchtmedien vereinzelt wird.

Vorzugsweise besitzt die Kolonie-Vereinzelungsvorrichtung eine Reinigungsvorrichtung zum Reinigen der Nadelspitze vor dem Sterilisieren. Das hat den Vorteil, dass etwaig vorhandene Organismen- oder Anzuchtsmediumsreste beim Sterilisieren nicht verkohlen und so die Vereinzelungsnadel verunreinigen oder solche Reste an der Vereinzelungsnadel verbleiben. Es hat sich jedoch herausgestellt, dass eine derartige Reinigungsvorrichtung in aller Regel nicht notwendig ist.

Günstig ist es, wenn die Kolonie-Vereinzelungsvorrichtung ein Gehäuse aufweist, das so ausgebildet ist, dass die zumindest eine Vereinzelungsnadel, die Haltevorrichtung und die Sterilisiervorrichtung vom Gehäuse umschließbar sind und dass die Kolonie-Vereinzelungsvorrichtung wie ein Koffer tragbar ist. Insbesondere hat das Gehäuse bezüglich zumindest zweier Raumrichtungen ein jeweiliges Ausmaß von höchstens einem Meter. In anderen Worten umschließt das Gehäuse alle übrigen Komponenten und bildet die äußere Hülle der Kolonie-Vereinzelungsvorrichtung. Es ist günstig, wenn das Gehäuse zumindest spritzwasserfest ist, so dass es zum Beispiel bei Feldversuchen mitgeführt werden kann.

Vorzugsweise aber nicht notwendigerweise, besitzt die Kolonie-Vereinzelungsvorrichtung eine Vorrichtung zur Einhaltung der sterilen Arbeitsumgebung im Arbeitsraum, insbesondere oberhalb des Trägers der Kolonien, oberhalb des Trägers des Anzuchtmediums sowie oberhalb der steril zu haltenden Komponenten der Kolonie-Vereinzelungsvorrichtung. Diese Vorrichtung umfasst vorzugsweise Filteranlagen und Ventilatoren oder entsprechend angebrachte Wärmequellen, beispielsweise Gasbrenner, zum Bewirken eines sterilen Luftstromes in den Arbeitsraum.

Günstig ist es, wenn der elektrische Strom für zumindest eine Sekunde angelegt wird. Beispielsweise beträgt die Zeit, während der der elektrische Strom angelegt ist, weniger als eine Minute.

Gemäß einer bevorzugten Ausführungsform besitzt die Kolonie-Vereinzelungsvorrichtung eine Aufnahme, die zum Aufnehmen der Haltevorrichtung ausgebildet ist, wobei die Elektrode so relativ zur Aufnahme angeordnet ist, die Nadelspitze mit der Elektrode Kontakt hat, wenn die Haltevorrichtung in der Aufnahme aufgenommen ist, und wobei die Stromversorgung so ausgebildet ist, dass elektrischer Strom durch Elektrode und Nadelspitze fließt, der die Vereinzelungsnadel zumindest abschnittsweise so stark erwärmt, dass sie sterilisiert wird, wenn die Haltevorrichtung in der Aufnahme aufgenommen ist. Es ist möglich, dass die Haltevorrichtung stiftförmig ist, so dass sie bequem mit der Hand gehalten werden kann. Wird die Haltevorrichtung dann in die Aufnahme eingesetzt, so kommt die Nadelspitze in Kontakt mit der Elektrode.

Es kann vorgesehen sein, dass die Kolonie-Vereinzelungsvorrichtung ein Kontaktelement besitzt, das mit einer Kontaktvorrichtung der Haltevorrichtung dann in Kontakt kommt, wenn die Haltevorrichtung in der Aufnahme aufgenommen ist. Es ist dann günstig, wenn die Stromversorgung ausgebildet ist zum regelmäßigen überprüfen, ob die Haltevorrichtung in der Aufnahme aufgenommen ist und bejahendenfalls zum Leiten des elektrischen Stroms durch die Nadelspitze sowie zum Unterbrechen des Stroms nach einer vorgegebenen Zeit. Alternativ ist möglich, dass zwischen der Elektrode und dem Kontaktelement stets eine Spannung anliegt, so dass die Nadelspitze sofort sterilisiert wird, wenn die Haltevorrichtung in die Aufnahme eingesetzt wird.

Günstig ist es, wenn der elektrische Strom so eingestellt wird, dass die Sterilisierungstemperatur der Vereinzelungsnadel oberhalb von 100° liegt, wobei eine Sterilisierungstemperatur von vorzugsweise unter 400° erreicht wird.

Im Folgenden wird die Erfindung anhand der beigefügten Zeichnungen näher erläutert. Dabei zeigt
- Figur 1: eine perspektivische Ansicht einer Kolonie-Vereinzelungsvorrichtung,
- Figur 2: eine zweite Ausführungsform einer erfindungsgemäßen Kolonie-Vereinzelungsvorrichtung in einer dreidimensionalen Ansicht,
- Figur 3: einen Revolver der Kolonie-Vereinzelungsvorrichtung in einer perspektivischen Ansicht und
- Figur 4: die Kolonie-Vereinzelungsvorrichtung gemäß der Figuren 2 und 3 in einer Explosionsansicht Und
- Figur 5: eine weitere Ausführungsform einer erfindungsgemäßen Kolonie-Vereinzelungsvorrichtung.

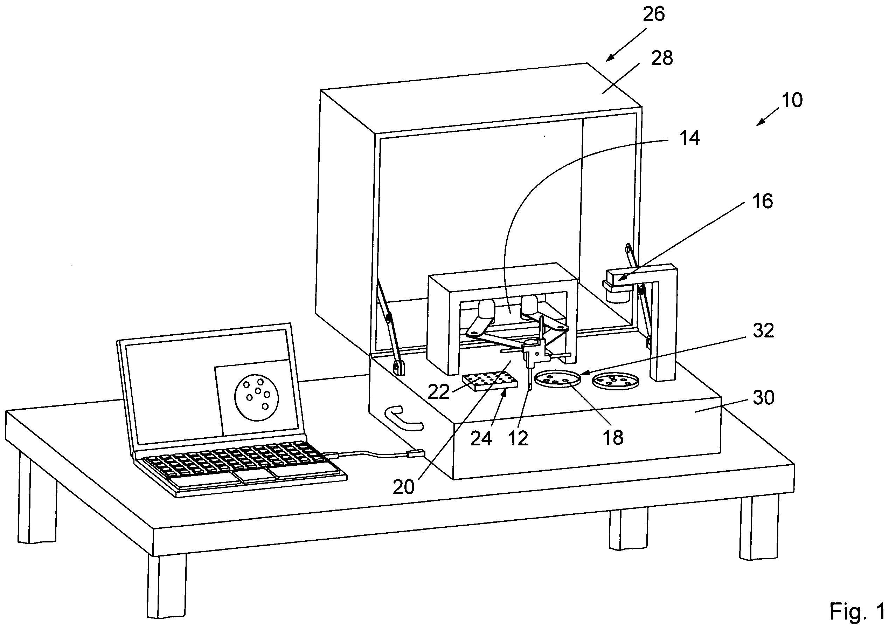

Figur 1 zeigt eine erfindungsgemäße Kolonie-Vereinzelungsvorrichtung 10, die eine Vereinzelungsnadel 12 und eine Positioniervorrichtung 14 zum Positionieren der Vereinzelungsnadel 12 aufweist. Die Kolonie-Vereinzelungsvorrichtung 10 besitzt zudem eine Kamera 16, mittels der die räumliche Lage einer Kolonie 18 erfassbar ist.

In einem Arbeitsraum 20, also dem Raum, der der Vereinzelungsnadel 12 zugänglich ist, ist ein Anzuchtmedium 22 auf einer Mikrotiterplatte 24 angeordnet. Die genannten Komponenten können von einem Gehäuse 26 umschlossen werden, das so ausgebildet ist, dass die Kolonie-Vereinzelungsvorrichtung 10 wie ein Koffer tragbar ist. Dazu besitzt das Gehäuse 26 einen Gehäusedeckel 28.

Im Betrieb wird eine Petrischale 32 mit der Kolonie 18 im Arbeitsraum 20 positioniert. Mit der Kamera 16 wird die Lage einer oder mehrerer zu übertragenden Kolonien 18 erfasst, sofern mehrere davon vorhanden sind. Die Positioniervorrichtung 14 positioniert nun die Vereinzelungsnadel 12 in der Kolonie 18 und anschließend auf dem Anzuchtmedium 22 und danach wird die Vereinzelungsnadel 12 desinfiziert.

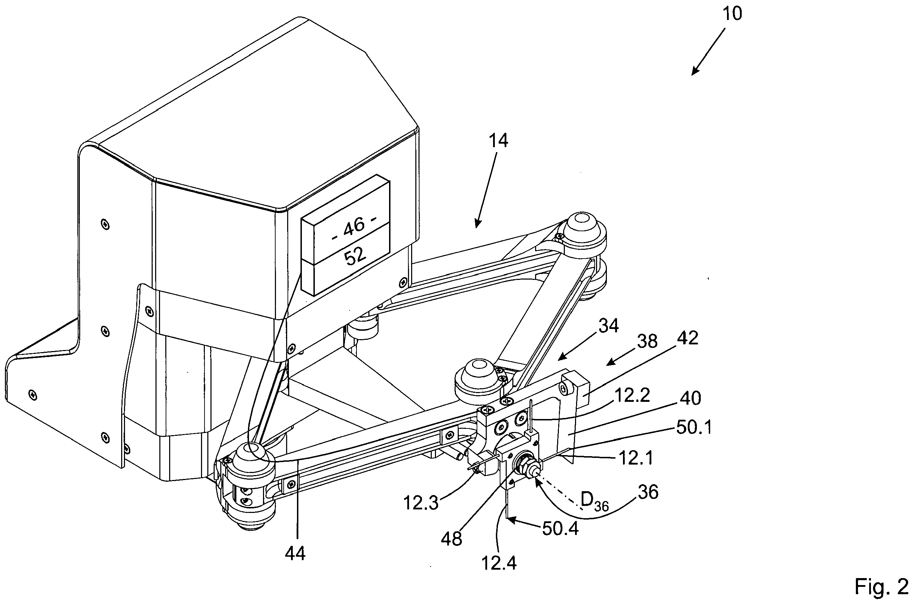

Figur 2 zeigt eine zweite Ausführungsform einer Kolonie-Vereinzelungsvorrichtung 10, die sich von der in Figur 1 gezeigten unter anderem dadurch unterscheidet, dass sie kein Gehäuse und keine Kamera aufweist. Die Kolonie-Vereinzelungsvorrichtung 10 besitzt eine Haltevorrichtung 34, die einen Revolver 36 umfasst. Am Revolver 36 sind vier Vereinzelungsnadeln 12.1, 12.2, 12.3, 12.4 befestigt. Die Kolonie-Vereinzelungsvorrichtung 10 besitzt zudem eine Sterilisiervorrichtung 38 zum automatischen Sterilisieren der Vereinzelungsnadeln 12 (Bezugszeichen ohne Zählsuffix bezeichnen das Objekt als solches), die in Figur 1 nicht eingezeichnet ist.

Die Sterilisiervorrichtung 38 umfasst eine Elektrode 40, im vorliegenden Fall in Form einer 0,05 mm dicken Metallfolie, die an einem Halter 42 befestigt und über diesen elektrisch kontaktiert ist. Der Halter 42 ist über eine schematisch eingezeichnete elektrische Leitung 44 mit einer schematisch eingezeichneten Stromversorgung 46 elektrisch verbunden.

Der Revolver 36 besitzt einen drehbaren Revolverkopf 48 der über eine nicht eingezeichnete zweite elektrische Leitung mit der Stromversorgung 46 so verbunden ist, dass über zumindest eine Vereinzelungsnadel 12, hier Vereinzelungsnadel 12.1, und der Elektrode 40 ein Stromkreis mit der Stromversorgung 46 geschlossen wird. Insbesondere die Nadelspitze der betreffenden Vereinzelungsnadel ist Bestandteil des Stromkreises. In diesem Stromkreis kann von der Stromversorgung 46 ein elektrischer Strom I bewirkt werden.

Figur 2 zeigt die Situation, in der die Elektrode 40 mit einer Nadelspitze 50.1 in Kontakt mit der Elektrode 40 steht. Es fließt ein elektrischer Strom I, beispielsweise ein Gleichstrom von 2 Ampere, von der Stromquelle zu der Elektrode 40, durch die Nadelspitze 50.1, durch den Rest der Vereinzelungsnadel 12.1, den Revolverkopf 48 und von dort zur Stromquelle 46. Aufgrund dieses elektrischen Stroms erwärmt sich die Nadelspitze 40 auf eine Sterilisierungstemperatur T_{S} von beispielsweise 150°. Nach einer Sterilisierzeit Δtₛ wird der Stromfluss unterbrochen, so dass die Temperatur T₅₀ der Nadelspitze 50 von der Sterilisiertemperatur T_{S} rasch auf Raumtemperatur T_{Raum} abfällt.

Die Elektrode 40 ist federnd ausgebildet. Wird daher der Revolverkopf 48 um eine Revolverdrehachse D₃₆ gedreht, so kommt die Nadelspitze 50.1 außer Kontakt mit der Elektrode 40 und nach einer Drehung von ungefähr 90° kommt die Nadelspitze 50.4 in elektrischen Kontakt mit der Elektrode 40. Da die Elektrode 40 federnd ausgebildet ist, weicht sie beim Kontakt mit der Nadelspitze 50.4 zurück, so dass weder die Elektrode 40 noch die Nadelspitze 50.4 beschädigt werden. Es ist günstig wenn, nicht aber notwendig, dass zu diesem Zeitpunkt noch kein Strom durch die Nadelspitze 50.4 fließt. Vorzugsweise ist in dem Stromkreis ein Sensor enthalten, beispielsweise eine Schaltung zur elektrischen Durchgangsprüfung, der den Zeitpunkt erkennt, ab dem der Kontakt hergestellt ist. Sobald eine schematisch eingezeichnete Steuereinheit 52 erfasst, dass der Revolverkopf 48 in seiner neuen Endposition angekommen ist, steuert die Steuereinheit 52 die Stromversorgungen 46 so an, dass die Nadelspitze 50.4 wie oben beschrieben sterilisiert wird.

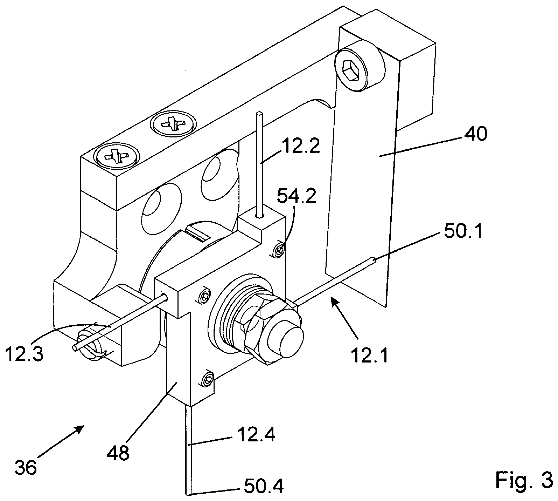

Figur 3 zeigt eine perspektivische Ansicht des Revolvers 36. Es ist zu erkennen, dass die Vereinzelungsnadeln 12.1, 12.2, 12.3, 12.4 lösbar am Revolverkopf 48 befestigt sind. So ist die Vereinzelungsnadel 12.2 mit einer Schraube 54.2 befestigt. Es ist unerheblich, ob die Nadeln lösbar sind oder nicht. Beides ist konstruktiv möglich und je nach Randbedingungen sinnvoll.

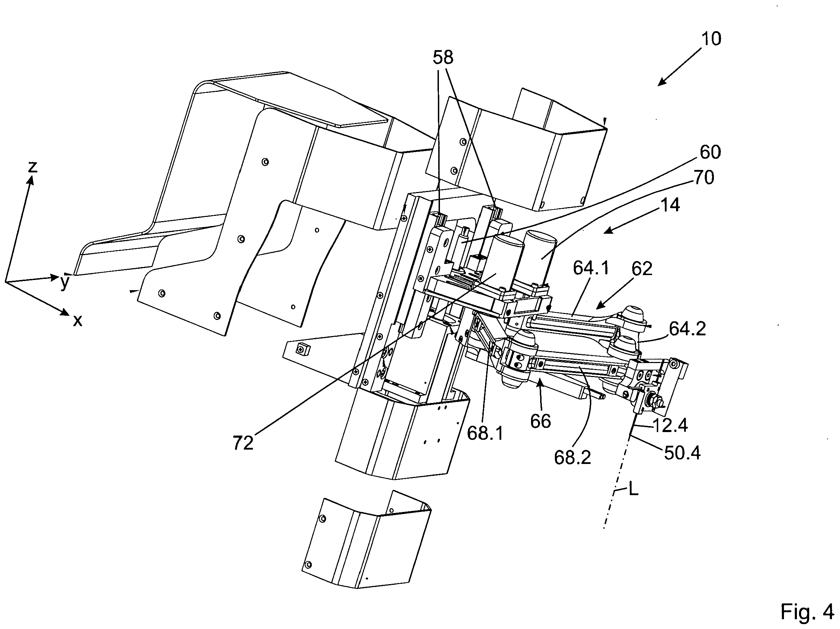

Figur 4 zeigt eine Explosionszeichnung der Kolonie-Vereinzelungsvorrichtung 10, die eine Längsführung 58 aufweist, mittels der die Vereinzelungsnadel 12, im vorliegenden Fall die Vereinzelungsnadel 12.4, bezüglich eines Bewegungsfreiheitsgrades entlang einer z-Achse positionierbar ist. Die z-Achse verläuft koaxial zu einer Längsachse L derjenigen Vereinzelungsnadel, im vorliegenden Fall der Vereinzelungsnadel 12.4, die als nächstes in die Kolonien 18 oder das Anzuchtmedium 22 getaucht werden soll. Die Haltevorrichtung 34 umfasst dazu einen Motor mit Spindeltrieb 60.

Die Positioniervorrichtung 14 besitzt zudem einen ersten Arm 62 mit einem ersten Armelement 64.1 und einem daran gelenkig angeordneten zweiten Armelement 64.2 sowie einen zweiten Arm 66 mit einem ersten Armelement 68.1 und einem zweiten Armelement 68.2. Die Arme 64.2 und 68.2 sind gelenkig miteinander verbunden. An einem der beiden Arme ist die Haltevorrichtung 34 angebracht. Der erste Arm 62 wird von einem ersten Armantrieb 70 betrieben der zweite Arm 66 von einem zweiten Armantrieb 72. Die beiden Armantriebe 70, 72 werden von der in Figur 4 nicht eingezeichneten Steuereinheit 52 angesteuert, so dass die Nadelspitze 50.4 in drei Dimensionen positionierbar ist.

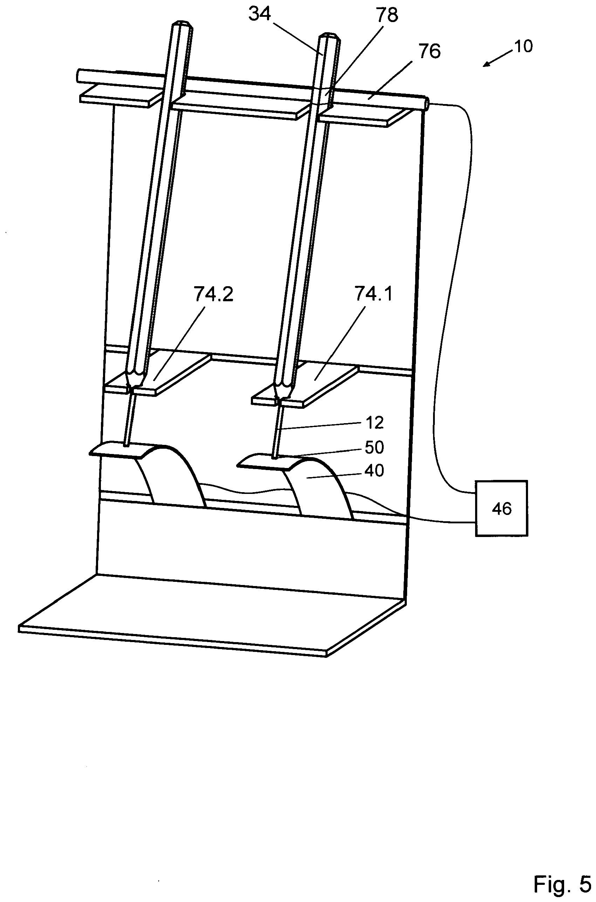

Figur 5 zeigt eine weitere Ausführungsform einer erfindungsgemäßen Kolonie-Vereinzelungsvorrichtung 10 mit der Haltevorrichtung 34, an der die Vereinzelungsnadel 12 befestigt ist. Die Kolonie-Vereinzelungsvorrichtung 10 umfasst eine erste Aufnahme 74.1 und eine zweite Aufnahme 74.2, in die die Haltevorrichtung 34 eingesetzt werden kann. Die Elektrode 40 ist als Feder ausgebildet und so zur Aufnahme 74.1 angeordnet, dass die Nadelspitze 50 mit der Elektrode 40 in Kontakt kommt, wenn die Haltevorrichtung 34 in der Aufnahme 74.1 aufgenommen ist. Die Kolonie-Vereinzelungsvorrichtung 10 besitzt ein Kontaktelement 76, das mit einer Kontaktvorrichtung 78 der Haltevorrichtung 34 dann in Kontakt kommt, wenn diese in der Aufnahme 74.1 aufgenommen ist. Die Haltevorrichtung 34 kann vollständig aus Metall bestehen. In diesem Fall ist die Kontaktvorrichtung 78 die Stelle, in der die Haltevorrichtung 34 in Kontakt mit dem Kontaktelement 76 kommt.

Die Stromversorgung 46 ist ausgebildet zum regelmäßigen überprüfen, ob die Haltevorrichtung 34 in der Aufnahme 74.1 aufgenommen ist. Ist das der Fall, leitet die Stromversorgung 46 elektrischen Strom durch die Nadelspitze 50, der durch die Vereinzelungsnadel 12, die Kontaktvorrichtung 78 und das Kontaktelement 76 fließt. Nach einer vorgegebenen Zeit wird der Strom unterbrochen.

### Bezugszeichenliste

- 10: Kolonie-Vereinzelungsvorrichtung
- 12: Vereinzelungsnadel
- 14: Positionsvorrichtung
- 16: Kamera
- 18: Kolonie

- 20: Arbeitsraum
- 22: Anzuchtmedium
- 24: Mikrotiterplatte
- 26: Gehäuse
- 28: Gehäusedeckel

- 30: Gehäuseboden
- 32: Petrischale
- 34: Haltevorrichtung
- 36: Revolver
- 38: Sterilisiervorrichtung

- 40: Elektrode
- 42: Halter
- 44: Leitung
- 46: Stromversorgung
- 48: Revolverkopf

- 50: Nadelspitze
- 52: Steuereinheit
- 54: Schraube
- 58: Längsführung

- 60: Motor mit Spindeltrieb
- 62: erster Arm
- 64: Armelement
- 66: zweiter Arm
- 68: Armelement

- 70: erster Armantrieb
- 72: zweiter Armantrieb
- 74: Aufnahme
- 76: Kontaktelement
- 78: Kontaktvorrichtung

- I: elektrischer Strom
- T_{S}: Sterilisierungstemperatur
- Δtₛ: Sterilisierzeit
- T₅₀: Temperatur
- D₃₆: Revolver-Drehachse

- L: Längsachse
- U: Spannung

## Patentansprüche

1. Kolonie-Vereinzelungsvorrichtung (10) mit
(a) zumindest einer Vereinzelungsnadel (12),
(b) einer Haltevorrichtung (34), an der die zumindest eine Vereinzelungsnadel (12) befestigt ist, und
(c) einer Sterilisiervorrichtung (38), die angeordnet ist zum Sterilisieren der Vereinzelungsnadel (12),
**dadurch gekennzeichnet, dass**
(d) die Sterilisiervorrichtung (38)
- eine Stromversorgung (46), die elektrisch mit der Vereinzelungsnadel (12) verbunden oder verbindbar ist, und
- eine Elektrode (40) aufweist,
(e) wobei die Elektrode (40) so mit einer Nadelspitze (50) der Vereinzelungsnadel (12) in Kontakt bringbar angeordnet ist, dass ein elektrischer Strom durch Elektrode (40) und Nadelspitze (50) fließt, der die Vereinzelungsnadel (12) zumindest abschnittsweise so stark erwärmt, dass sie zumindest abschnittsweise sterilisiert wird.

2. Kolonie-Vereinzelungsvorrichtung (10) nach Anspruch 1, **gekennzeichnet durch** eine Positioniervorrichtung (14), mittels der die Vereinzelungsnadel (12) bezüglich zumindest eines Freiheitsgrads positionierbar ist.

3. Kolonie-Vereinzelungsvorrichtung (10) nach einem der vorstehenden Ansprüche, **dadurch gekennzeichnet, dass** die Haltevorrichtung (34) einen Revolver (36) oder eine andere Schwenkeinrichtung umfasst.

4. Kolonie-Vereinzelungsvorrichtung (10) nach einem der vorstehenden Ansprüche, **dadurch gekennzeichnet, dass** die Elektrode (40) federnd ausgebildet und/oder gelagert ist.

5. Kolonie-Vereinzelungsvorrichtung (10) nach einem der vorstehenden Ansprüche, **gekennzeichnet durch** eine Steuereinheit (52), die ausgebildet ist zum automatischen Durchführen eines Verfahrens mit den Schritten:
(i) Positionieren der Vereinzelungsnadel (12), so dass die Nadelspitze (50) in eine Kolonie (18) eintaucht,
(ii) danach Positionieren der Nadelspitze (50) in einem Anzuchtmedium (22), so dass das Anzuchtmedium (22) mit den Organismen der Kolonie angeimpft wird,
(iii) danach In-Kontakt-Bringen der Nadelspitze (50) mit der Elektrode (40),
(iv) Bewirken von elektrischem Strom (I), der durch die Elektrode (40) und die Nadelspitze (50) fließt, so dass sich die Vereinzelungsnadel (12) zumindest im Bereich der Nadelspitze (50) auf eine Sterilisierungstemperatur (T_{S}) erwärmt, und
(v) nach einer vorgegebenen Sterilisierzeit (Δtₛ) Vermindern oder Unterbinden des Stromflusses durch die Vereinzelungsnadel (12), so dass die Vereinzelungsnadel (12) abkühlt.

6. Kolonie-Vereinzelungsvorrichtung (10) nach einem der vorstehenden Ansprüche, **dadurch gekennzeichnet, dass** die Positioniervorrichtung (14) ausgebildet ist zum Positionieren der Nadelspitze (50) auf eine vorgebbare Raumposition.

7. Kolonie-Vereinzelungsvorrichtung (10) nach einem der vorstehenden Ansprüche, **gekennzeichnet durch** ein Gehäuse (26), das so ausgebildet ist, dass die zumindest eine Vereinzelungsnadel (12), die Haltevorrichtung (34) und die Sterilisiervorrichtung (38) vom Gehäuse (26) umschließbar sind und dass die Kolonie-Vereinzelungsvorrichtung (10) wie ein Koffer tragbar ist.

8. Kolonie-Vereinzelungsvorrichtung (10) nach einem der vorstehenden Ansprüche, **gekennzeichnet durch**
(a) zumindest eine Aufnahme (74.1), die zum Aufnehmen der Haltevorrichtung (34) ausgebildet ist,
(b) wobei die Elektrode (40) so relativ zur zumindest einen Aufnahme angeordnet ist, die Nadelspitze (50) mit der Elektrode (40) Kontakt hat, wenn die Haltevorrichtung in der Aufnahme (74.1) aufgenommen ist, und
(c) wobei die Stromversorgung (46) so ausgebildet ist, dass elektrischer Strom durch Elektrode (40) und Nadelspitze (50) fließt, der die Vereinzelungsnadel (12) zumindest abschnittsweise so stark erwärmt, dass sie sterilisiert wird, wenn die Haltevorrichtung (34) in der Aufnahme (74.1) aufgenommen ist.

9. Verfahren zum Vereinzeln von Bakterienkolonien, mit den Schritten:
(i) Positionieren einer Vereinzelungsnadel (12) einer Kolonie-Vereinzelungsvorrichtung (10), so dass eine Nadelspitze (50) der Vereinzelungsnadel (12) in eine Kolonie (18) ragt,
(ii) danach Positionieren der Nadelspitze (50) in einem Anzuchtmedium (22), so dass Organismen der Kolonie auf das Anzuchtmedium (22) übertragen werden,
(iii) danach In-Kontakt-Bringen der Nadelspitze (50) mit einer Elektrode (40) der Kolonie-Vereinzelungsvorrichtung (10),
(iv) Bewirken eines elektrischen Stroms durch Elektrode (40) und Nadelspitze (50) in die Vereinzelungsnadel (12), so dass sich zumindest der Bereich um die Nadelspitze (50) auf eine Sterilisierungstemperatur (T_{S}) erwärmt, und
(v) nach einer vorgegebenen Sterilisierzeit (Δtₛ) Vermindern oder Unterbinden des Stromflusses durch die Vereinzelungsnadel (12), so dass die Nadelspitze (50) abkühlt.

10. Sterilisiervorrichtung (38) für elektrisch leitende Nadeln aus Vollmaterial, also keine Hohlnadeln oder Kanülen, beispielsweise eine Vereinzelungsnadel, **dadurch gekennzeichnet, dass**
(a) eine Nadel mit dem einen Pol einer Stromversorgung verbunden oder verbindbar ist,
(b) eine Elektrode (40) mit dem anderen Pol der Stromversorgung (46) verbunden oder verbindbar ist,
(c) die Elektrode (40) mit der Nadelspitze (50) in Kontakt bringbar angeordnet ist, so dass durch den damit geschlossenen Stromkreis ein elektrischer Strom (I) durch Elektrode (40) und Nadelspitze (50) fließen kann, der die Nadel zumindest abschnittsweise so stark erwärmt, dass sie aufgrund der hohen Temperatur zumindest abschnittsweise, vorzugsweise an der Nadelspitze (50), sterilisiert wird.

11. Sterilisiervorrichtung (38) nach einem der vorstehenden Ansprüche, **gekennzeichnet durch** eine Sensoranordnung beispielsweise eine elektrische Schaltung zur Durchgangsprüfung, die mit der Sterilisierungseinheit so verbunden ist, dass der Kontaktschluss der Vereinzelungsnadel (12) mit der Elektrode (40) erkannt wird und die Bewirkung des Stromes (I) zur Sterilisierung steuert.

## Claims

1. A colony isolation device (10) with
(a) at least one (12) isolation needle (12),
(b) a holding device (34) to which the at least one isolation needle (12) is attached, and
(c) a sterilisation device (38) for sterilising the isolation needle (12), **characterised by** the fact that
(d) the sterilisation device (38) has
- a power supply (46) which
is electrically connected or can be electrically connected to the isolation needle (12), and
- an electrode (40),
(e) wherein the electrode (40) is arranged so that it can be brought into contact with a needle tip (50) of the isolation needle (12), resulting in the flow of an electric current through the electrode (40) and the needle tip (50); this current at least partially heats the isolation needle (12) to such an extent that it is at least partially sterilised.

2. The colony isolation device (10) according to claim 1, **characterised by** a positioning device (14) by means of which the isolation needle (12) can be positioned relative to at least one degree of freedom.

3. The colony isolation device (10) according to one of the above claims, **characterised by** the fact that the holding device (34) comprises a turret (36) or another swivel mechanism.

4. The colony isolation device (10) according to one of the above claims, **characterised by** the fact that the electrode (40) is designed and/or stored such that it is spring-mounted.

5. The colony isolation device (10) according to one of the above claims, **characterised by** a control unit (52) that is designed to automatically carry out a method featuring the steps:
(i) positioning of the isolation needle (12) so the needle tip (50) is immersed in a colony (18),
(ii) the subsequent positioning of the needle tip (50) in a cultivation medium (22) such that the cultivation medium (22) is inoculated with the organisms of the colony,
(iii) the subsequent establishment of contact between the needle tip (50) and the electrode (40),
(iv) introduction of an electric current (I) which flows through the electrode (40) and the needle tip (50) so that the isolation needle (12) heats up to a sterilisation temperature (Ts) at least in the area around the needle tip (50), and
(v) reduction or suppression of the flow of electricity through the isolation needle (12) after a pre-set sterilisation time (Δtₛ), thereby causing the isolation needle (12) to cool down.

6. The colony isolation device (10) according to one of the above claims, **characterised by** the fact that the positioning device (14) is designed to move the needle tip (50) into an adjustable position.

7. The colony isolation device (10) according to one of the above claims, **characterised by** a case (26) which is designed in such a way that the at least one isolation needle (12), the holding device (34) and the sterilisation device (38) can be enclosed by the case (26) and the colony isolation device (10) can be carried like a suitcase.

8. The colony isolation device (10) according to one of the above claims, **characterised by**
(a) at least one accommodation (74.1) that is designed to accommodate the holding device (34),
(b) wherein the electrode (40) is arranged relative to at least one accommodation in such a way that the needle tip (50) is in contact with the electrode (40) when the holding device is in the accommodation (74.1), and
(c) wherein the power supply (46) is designed in such a way that an electric current flows through the electrode (40) and the needle tip (50), heating the isolation device (12) at least partially to such an extent that it is sterilised when the holding device (34) is in the accommodation (74.1).

9. A method for isolating bacterial colonies with the steps:
(i) positioning of an isolation needle (12) of a colony isolation device (10) such that a needle tip (50) of the isolation needle (12) projects into a colony (18),
(ii) the subsequent positioning of the needle tip (50) in a cultivation medium (22) such that organisms of the colony are transferred to the cultivation medium (22),
(iii) the subsequent establishment of contact between the needle tip (50) and the electrode (40) of the colony isolation device (10),
(iv) introduction of an electric current through the electrode (40) and the needle tip (50), and into the isolation needle (12) such that at least the area around the needle tip (50) heats up to a sterilisation temperature (Ts), and
(v) reduction or suppression of the flow of electricity through the isolation needle (12) after a pre-set sterilisation time (Δtₛ), thereby causing the needle tip (50) to cool down.

10. A sterilisation device (38) for electroconductive needles made of solid material, i.e. not hollow needles or tubes, for example an isolation needle, **characterised by** the fact that
(a) a needle is connected or can be connected to one pole of a power supply,
(b) an electrode (40) is connected or can be connected to the other pole of a power supply (4),
(c) the electrode (40) is arranged such that it can be brought into contact with the needle tip (50), thereby completing an electrical circuit and enabling an electric current (I) to flow through the electrode (40) and the needle tip (50); the current at least partially heats the needle to such an extent that, as a result of the high temperature, it is at least partially sterilised, preferably at the needle tip (50).

11. A sterilisation device (38) according to one of the above claims, **characterised by** a sensor arrangement, such as an electrical circuit for continuity testing, that is connected to the sterilisation unit in such a way that the establishment of contact between the isolation needle (12) and the electrode (40) is identified and the supply of the current (I) for sterilisation is controlled.

## Revendications

1. Dispositif (10) d'individualisation de colonies, comportant
(a) au moins une aiguille d'individualisation (12),
(b) un dispositif de maintien (34) sur lequel ladite au moins une aiguille d'individualisation (12) est fixée, et
(c) un dispositif de stérilisation (38) qui est agencé pour stériliser l'aiguille d'individualisation (12),
**caractérisé en ce que**
(d) le dispositif de stérilisation (38) comprend
- une alimentation électrique (46)
qui est connectée ou susceptible d'être connectée électriquement à l'aiguille d'individualisation (12), et
- une électrode (40),
(e) l'électrode (40) étant agencée de manière à pouvoir être mise en contact avec une pointe (50) de l'aiguille d'individualisation (12) de telle sorte qu'un courant électrique circule à travers l'électrode (40) et la pointe (50) de l'aiguille, qui échauffe l'aiguille d'individualisation (12) au moins localement si fortement qu'elle est stérilisée au moins localement.

2. Dispositif (10) d'individualisation de colonies selon la revendication 1, **caractérisé par** un dispositif de positionnement (14) permettant de positionner l'aiguille d'individualisation (12) par rapport à au moins un degré de liberté.

3. Dispositif (10) d'individualisation de colonies selon l'une des revendications précédentes, **caractérisé en ce que** le dispositif de maintien (34) comprend une tourelle (36) ou un autre moyen de pivotement.

4. Dispositif (10) d'individualisation de colonies selon l'une des revendications précédentes, **caractérisé en ce que** l'électrode (40) est réalisée et/ou montée de façon élastique.

5. Dispositif (10) d'individualisation de colonies selon l'une des revendications précédentes, **caractérisé par** une unité de commande (52) qui est réalisée pour mettre en oeuvre automatiquement un procédé comprenant les étapes consistant à :
(i) positionner l'aiguille d'individualisation (12) de telle sorte que la pointe (50) de l'aiguille vient plonger dans une colonie (18),
(ii) puis positionner la pointe (50) de l'aiguille dans un milieu de culture (22) de telle sorte que le milieu de culture (22) est inoculée avec les organismes de la colonie,
(iii) puis mettre en contact la pointe (50) de l'aiguille avec l'électrode (40),
(iv) appliquer un courant électrique (I) qui circule à travers l'électrode (40) et la pointe (50) de l'aiguille, de sorte que l'aiguille d'individualisation (12) s'échauffe à une température de stérilisation (Ts) au moins dans la zone de la pointe (50) de l'aiguille, et
(v) après une durée de stérilisation donnée (Δtₛ), réduire ou interrompre la circulation du courant à travers l'aiguille d'individualisation (12), de sorte que l'aiguille d'individualisation (12) se refroidit.

6. Dispositif (10) d'individualisation de colonies selon l'une des revendications précédentes, **caractérisé en ce que** le dispositif de positionnement (14) est réalisé pour positionner la pointe (50) de l'aiguille dans une position spatiale susceptible d'être prédéterminée.

7. Dispositif (10) d'individualisation de colonies selon l'une des revendications précédentes, **caractérisé par** un boîtier (26) qui est réalisé de telle sorte que ladite au moins une aiguille d'individualisation (12), le dispositif de maintien (34) et le dispositif de stérilisation (38) peuvent être enfermés par le boîtier (26), et que le dispositif (10) d'individualisation de colonies est portatif comme une mallette.

8. Dispositif (10) d'individualisation de colonies selon l'une des revendications précédentes, **caractérisé par**
(a) au moins un logement (74.1) qui est réalisé pour loger le dispositif de maintien (34),
(b) l'électrode (40) étant agencé par rapport audit au moins un logement de telle sorte que la pointe (50) de l'aiguille est en contact avec l'électrode (40) lorsque le dispositif de maintien est logé dans le logement (74.1), et
(c) l'alimentation électrique (46) est réalisée de telle sorte qu'un courant électrique circule à travers l'électrode (40) et la pointe (50) de l'aiguille qui échauffe l'aiguille d'individualisation (12) au moins localement si fortement qu'elle est stérilisée lorsque le dispositif de maintien (34) est logé dans le logement (74.1).

9. Procédé d'individualisation de colonies bactériennes, comprenant les étapes consistant à :
(i) positionner une aiguille d'individualisation (12) d'un dispositif (10) d'individualisation de colonies de telle sorte qu'une pointe (50) de l'aiguille vient pénétrer dans une colonie (18),
(ii) puis positionner la pointe (50) de l'aiguille dans un milieu de culture (22) de telle sorte que des organismes de la colonie sont transférés au milieu de culture (22),
(iii) puis mettre en contact la pointe (50) de l'aiguille avec une électrode (40) du dispositif (10) d'individualisation de colonies,
(iv) appliquer un courant électrique (I) à travers l'électrode (40) et la pointe (50) de l'aiguille dans l'aiguille d'individualisation (12), de sorte qu'au moins la zone autour de la pointe (50) de l'aiguille s'échauffe à une température de stérilisation (Ts), et
(v) après une durée de stérilisation donnée (Δtₛ), réduire ou interrompre la circulation du courant à travers l'aiguille d'individualisation (12), de sorte que la pointe (50) de l'aiguille se refroidit.

10. Dispositif de stérilisation (38) pour des aiguilles électriquement conductrices en matériau massif, donc non pas pour des aiguilles creuses ou des canules, par exemple pour une aiguille d'individualisation, **caractérisé en ce que**
(a) une aiguille est connectée ou susceptible d'être connectée à un pôle d'une alimentation électrique,
(b) une électrode (40) est connectée ou susceptible d'être connectée à l'autre pôle de l'alimentation électrique (46),
(c) l'électrode (40) est agencée de manière à pouvoir être mise en contact avec la pointe (50) de l'aiguille, de telle sorte que par le circuit électrique ainsi fermé, un courant électrique (I) peut circuler à travers l'électrode (40) et la pointe (50) de l'aiguille, qui échauffe l'aiguille au moins localement si fortement qu'elle est stérilisée au moins localement, de préférence à la pointe (50) de l'aiguille, en raison de la température élevée.

11. Dispositif de stérilisation (38) selon l'une des revendications précédentes, **caractérisé par** un agencement capteur, par exemple un circuit électrique pour le contrôle de la continuité électrique, qui est connecté à l'ensemble de stérilisation de telle sorte que la mise en contact de l'aiguille d'individualisation (12) avec l'électrode (40) est reconnue et commande l'application du courant (I) pour la stérilisation.
